# NEUE EUROPÄISCHE PATENTSCHRIFT

(11) **EP 1 197 199 B2**
(45) Veröffentlichungstag und Bekanntmachung des Hinweises auf die Entscheidung über den Einspruch: **28.05.2008**
(45) Hinweis auf die Patenterteilung: 12.01.2005
(21) Anmeldenummer: 01123311.1
(22) Anmeldetag: 08.10.2001
(51) Int. Cl.: A61K 8/37, A61K 8/39, A61K 8/81, A61Q 5/06

(54) **Haarbehandlungsmittel**
Hair care product
Produit pour le soin des cheveux

(30) Priorität: 12.10.2000 DE 10050452
(43) Veröffentlichungstag der Anmeldung: 17.04.2002
(73) Patentinhaber: KPSS-Kao Professional Salon Services GmbH, 64297 Darmstadt (DE)
(72) Erfinder: Hoffmann, Martin, 64673 Zwingenberg (DE)

(56) Entgegenhaltungen:
- EP-A- 0 422 862
- DE-A- 119 914 927
- US-A- 5 364 625
- US-A- 5 972 322
- RHEOCARE ATH, [Online] XP002276713 Gefunden im Internet: URL:http://www.cosrheo.com/ath.html> [gefunden am 2004-03-17]
- COSMETICS AND TOILETRIES, Bd., 105, Dezember 1990,Seite 129
- COSMETICS AND TOILETRIES, Bd. 105, Dezember 1990, Seite 92
- THE BF GOODRICH COMPANY: "Polymers for Personal Care"
- K. SCHRADER: "Grundlagen und Rezepturen der Kosmetik" 1979, HÜTHIG VERLAG; Seiten 500/501, 515/516
- "Conditioning Setting Gel, DC 954 Formulation Sheet number 22-1269" August 1989
- "After Bath Lotion, DC 949, Formulation Sheet number 22-1274" August 1989
- EUROPEAN FORMULARY GUIDE, 1999, SEITE 4

## Beschreibung

Die vorliegende Erfindung betrifft ein stabiles, einfach herstellbares Haarbehandlungsmittel in Form einer Öl-in-Wasser-Emulsion, das den Haaren eine verbesserte Naß- und Trockenkämmbarkeit, Volumen, Spannkraft, dezenten Glanz und insbesondere Frisur-modellierende Eigenschaften verleiht.

Solche Mittel enthalten seit langem auch Wirkstoffe mit haarkonditionierenden Eigenschaften zur Verbesserung der Eigenschaften des damit behandelten Haares.

Für diesen Zweck ist eine Vielzahl der unterschiedlichsten Wirkstoffe vorgeschlagen worden, von fett- bzw. ölartigen synthetischen und natürlichen Stoffen, Proteinen und deren Abbauprodukten, modifiziert oder nicht, über natürliche und synthetische Polymere, insbesondere kationische Polymere, bis hin zu langkettigen quaternären Ammoniumverbindungen.

Es besteht jedoch nach wie vor ein Bedürfnis an haarkonditionierenden Haarpflegeund Haarbehandlungsmitteln, insbesondere in Form von Öl-in-Wasser-Emulsionen, die auch stabil und leicht herstellbar sind.

Es wurde nun gefunden, und das ist Gegenstand der vorliegenden Erfindung, daß ein solches Mittel zum Pflegen und Behandeln von menschlichen Haaren dann erhalten wird, wenn dieses eine Kombination aus
a) mindestens einem anionischen, nichtionischen, kationischen und/oder zwitterionischen bzw. amphoteren Polymeren und
b) einem Gemisch aus einem Salz eines Acrylsäure-Polymeren, einem C₃-C₁₀-Alkyl-C₁₂-C₁₈-fettsäureester und einem C₁₂-C₂₄-Fettalkoholethoxylat ausgewählt aus Trideceth-3, Trideceth-6, Laureth-4, Laureth-8, Myristeth-4, Myristeth-6, Ceteth-2 und Oleth-5 enthält.

Bevorzugter Bestandteil b) ist ein Gemisch aus einem Salz eines Homo- oder Copolymeren der Acrylsäure, insbesondere ein Ammonium-, Natrium-, Kaliumund/oder Aminsalz desselben, einem C₁₄-C₁₈-Fettsäure-C₄-C₁₀-alkylester, insbesondere Ethylhexylstearat, n-Butyl, n-Propyl und Isopropylmyristat, -stearat oder -isostearat, und einem C₁₂-C₁₆-Fettalkoholethoxylat, insbesondere mit 2 bis 10 Ethylenoxidgruppen pro Molekül, ausgewahlt aus Trideceth-3, Trideceth-6, Laureth-4, Laureth-8, Myristeth-4, Myristeth-6, Ceteth-2 oder Oleth-5.

Der Feststoffgehalt dieser Polyacrylsäuresalz-Dispersion liegt vorzugsweise zwischen etwa 30 und etwa 75, insbesondere etwa 40 bis etwa 65 Gew.-%.

Ein besonders geeignetes Produkt ist die unter dem Handelsnamen "RHEOCARE^{R} ATH" bekannte Zusammensetzung, vorzugsweise in einer Menge von etwa 0,1 bis etwa 5, insbesondere etwa 0,5 bis etwa 2,5 Gew.-% der Öl-in-Wasser-Emulsion, bezogen auf den Fettstoffgehalt.

Der zweite essentielle Bestandteil der erfindungsgemäßen Zusammensetzung ist mindestens ein anionisches, nichtionisches, kationisches und/oder amphoteres bzw. zwitterionisches natürliches oder synthetisches Polymer, vorzugsweise in einer Menge von etwa 0,1 bis 5, insbesondere 0,5 bis 2,5 Gew.-% der Öl-in-Wasser-Emulsion.

Bevorzugte Polymere im Rahmen der Erfindung sind nichtionische Polymere, vorzugsweise alkohol- und/oder wasserlösliche Vinylpyrrolidon-Polymere wie ein Vinylpyrrolidon-Homopolymerisat oder -Copolymerisat, insbesondere mit Vinylacetat. Geeignete Vinylpyrrolidon-Polymere sind z.B. die unter dem Handelsnamen "Luviskol®" bekannten Produkte, beispielsweise die Homopolymerisate "Luviskol® K 30, K 60 und K 90" sowie die wasser- bzw. alkohollöslichen Copolymerisate aus Vinylpyrrolidon und Vinylacetat, die unter dem Handelsnamen "Luviskol® VA 55 bzw. VA 64" von der BASF AG vertrieben werden.

Weitere geeignete nichtionische Polymere sind Vinylpyrrolidon/Vinylacetat/Vinylpropionat-Copolymere wie "Luviskol® VAP 343", Vinylpyrrolidon/(Meth) Acrylsäureester-Copolymere sowie Chitosan-Derivate.

Anstelle oder zusätzlich zu den nichtionischen Polymeren können als konditionierende Polymere insbesondere anionische und/oder amphotere Polymere in den genannten Mengen eingesetzt werden.

Geeignete anionische Polymere sind Vinylalkylether-, insbesondere Methylvinylether/Maleinsäure-Copolymere, die durch Hydrolyse von Vinylether/Malein säureanhydrid-Copolymeren entstehen und unter der Handelsbezeichnung "Gantrez® AN oder ES" vertrieben werden. Diese Polymeren können auch teilverestert sein, beispielsweise "Gantrez® ES 225", der Ethylester eines Ethylvinylethers/Maleinsäure-Copolymers, oder der Butyl- oder Isobutylester desselben.

Weitere geeignete anionische Polymere sind insbesondere Vinylacetat/Crotonsäureoder Vinylacetat/Vinylneodecanoat/Crotonsäure-Copolymere des Typs "Resyn®"; Natriumacrylat/Vinylalkohol-Copolymere des Typs "Hydagen® F", Natriumpolystyrolsulfonat, z.B. "Flexan® 130"; Ethylacrylat/Acrylsäure/N-tert.-Butylacrylamid-Copolymere des Typs "Ultrahold®"; Vinylpyrrolidon/Vinylacetat/Itaconsäure-Copolymere, Acrylsäure/Acrylamid-Copolymere bzw. Natriumsalze derselben vom Typ "Reten®"; etc.

Als amphotere Polymere, die allein oder im Gemisch mit mindestens einem weiteren kationischen, nichtionischen oder anionischen Polymeren zum Einsatz gelangen, seien insbesondere Copolymerisate aus N-Octylacrylamid, (Meth)Acrylsäure und tert.-Butylaminoethylmethacrylat vom Typ "Amphomer®"; Copolymerisate aus Methacryloylethylbetain und Alkylmethacrylaten vom Typ "Yukaformer®", z. B. das Butylmethacrylat-Copolymere "Yukaformer® Am75"; Copolymerisate aus Carboxylgruppen und Sulfongruppen enthaltenden Monomeren, z. B. (Meth)Acrylsäure und Itaconsäure, mit basische Gruppen, insbesondere Aminogruppen, enthaltenden Monomeren wie Mono- bzw. Dialkylaminoalkyl(meth)acrylaten bzw. Mono- bzw. Dialkylaminoalkyl(meth)acrylamiden; Copolymere aus N-Octylacrylamid, Methylmethacrylat, Hydroxypropylmethacrylat, N-tert.-Butylaminoethylmethacrylat und Acrylsäure sowie die aus der US-A 3,927,199 bekannten Copolymeren genannt.

Bevorzugte konditionierende kationische Polymere sind die altbekannten quaternären Cellulosederivate des Typs "Polymer JR" sowie quaternisierte Homound Copolymere des Dimethyldiallylammoniumchlorids, wie sie unter dem Handelsnamen "Merquat®" im Handel sind, quaternäre Vinylpyrrolidon-Copolymere, insbesondere mit Dialkylaminoalkyl(meth)acrylaten, wie sie unter dem Namen "Gafquat®" bekannt sind, Copolymerisate aus Vinylpyrrolidon und Vinylimidazoliniummethochlorid, die unter dem Handelsnamen "Luviquat®" angeboten werden, Polyamino-Polyamid-Derivate, beispielsweise Copolymere von Adipinsäure-Dimethylaminohydroxypropyldiethylentriamin, wie sie unter dem Namen "Cartaretine® F" vertrieben werden, sowie auch bisquaternäre langkettige Ammoniumverbindungen der in der US-PS 4 157 388 beschriebenen Harnstoff-Struktur, die unter dem Handelsnamen "Mirapol® A 15" erhältlich sind.
Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymerer eingesetzt werden.
Zu den kationischen Polymeren zählen auch die in der EP-A 524 612 und der EP-A 640 643 beschriebenen Quaternisierungsprodukte aus Pfropfpolymerisaten von Organopolysiloxanen und Polyethyloxazolinen.

Die erfindungsgemäßen Mittel können selbstverständlich alle in diesen üblichen Stoffe enthalten.

Als solche seien beispielhaft Komplexbildner, Farbstoffe, Konservierungsmittel, pH-Regler, Viskositätsregler, Duftstoffe, Perlglanzmittel, Verdickungsmittel, Feuchthaltemittel, pflanzliche und tierische Öle wie Jojobaöl, Fettsäureester wie z.B. Isopropylmyristat, Ethylpalmitat, Lecithin und dessen Derivate etc. genannt. Eine Auflistung solcher Zusatzstoffe findet sich in der Monographie von K. Schrader, "Grundlagen und Rezepturen der Kosmetika", 2. Aufl. (1989) S. 695 bis 762.
Geeignete Fette und Öle, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline.
Synthetische Öle und Wachse sind beispielsweise Polyethylenglykole.
Weitere geeignete hydrophobe Komponenten sind insbesondere Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat, Cetylpalmitat, etc.
Diese hydrophoben Komponenten sind in den erfindungsgemäßen Zusammensetzungen vorzugsweise in einer Gesamtmenge von etwa 1 bis etwa 25, insbesondere etwa 2,5 bis 20, vor allem etwa 5 bis 15 Gew.-%, berechnet auf die Gesamtzusammensetzung, enthalten.

Die erfindungsgemäßen Mittel können auch langkettige Fettsäuren enthalten.
Als Fettsäuren werden bevorzugt solche mit 10 bis 24, insbesondere 12 bis 22 Kohlenstoffatomen in einer Menge von etwa 0,5 bis 15 Gew.-%, insbesondere 1 bis 10 Gew.-%, bezogen auf die Gesamtzusammensetzung, verwendet. Besonders geeignet sind Behensäure und Stearinsäure; jedoch können auch andere Fettsäuren wie beispielsweise Myristinsäure, Palmitinsäure oder Ölsäure oder auch Gemische natürlicher oder synthetischer Fettsäuren wie Kokosfettsäure eingesetzt werden.

Ein weiterer bevorzugter Bestandteil in Haarbehandlungsmitteln ist Harnstoff, vorzugsweise in einer Menge von etwa 1 bis 10, insbesondere etwa 2,5 bis 7,5 Gew.-% des erfindungsgemäßen Mittels.

Das erfindungsgemäße Haarbehandlungsmittel kann als weiteren Bestandteil noch mindestens eine Verbindung, ausgewählt aus der Gruppe 1-Methoxypropanol(-2), 1-Ethoxypropanol(-2), Diethylenglykolmonomethyl- oder ethylether, Dipropylenglykolmonomethyl- oder -ethylether, Benzylalkohol, Benzyloxyethanol, Phenylethylalkohol, Phenoxyethanol und/oder Zimtalkohol, vorzugsweise in einer Menge von 0,5 bis 25, insbesondere 1 bis 20, vor allem 2,5 bis 15 Gew.-%, berechnet auf die Gesamtzusammensetzung des Mittels, enthalten.
Bevorzugte Verbindungen aus dieser Gruppe sind Ethoxydiglykol und Benzyloxyethanol.

Die erfindungsgemäßen Mittel können selbstverständlich auch übliche O/W-Emulgatoren enthalten.
Diese sind dem Fachmann bekannt, und auch bei Schrader, I.c., S. 390 bis 424, in allgemeiner Form beschrieben.

Die Herstellung der erfindungsgemäßen W/O-Emulsionen erfolgt durch einfaches Zusammenrühren der Bestandteile, was durch die Kombination der jeweiligen essentiellen Bestandteile ermöglicht wird.

Die folgenden Beispiele illustrieren die Erfindung:

### Beispiel 1

### Glanz-Modelling-Creme

4,0 kg Capryl-/Caprinsäuretriglycerid, 3,6 kg Cetearyloctanoat, 0,4 kg Isopropylmyristat, 2,0 kg Mineralöl und 0,3 kg Parfumöl wurden unter Rühren bei 20rpm vermischt und anschließend mit einer wäßrigen Phase aus 3,0 kg 1,2-Propandiol, 2,0 kg Vinylpyrrolidon/Vinyllacetat-Copolymerisat (Luviskol^{R}VA 37 I), 3,0 kg Glycerin und 0,3 kg Konservierungsmittel sowie 2,0 kg "Rheocare^{R} ATH" in 81,4 kg Wasser bei 25rpm 10 Minuten verrührt.

Es wird eine stabile O/W-Emulsion mit ausgezeichneten haarkonditionierenden Eigenschaften erhalten, die sich insbesondere zur charakteristischen Frisur-Modellierung eignet.

### Beispiel 2

### Haarkur (O/W-Emulsion)

| | |
|---|---|
| Paraffinöl | 10,0 (Gew.-%) |
| Rosa Mikroteilchen aus Kokosöl, Ceramid und | 1,0 |
| Avocadin / Fa. Lipotec (mittl. Teilchengröße~2000µm) | |
| Rheocare^{R} ATH | 2,0 |
| Kaliumhydroxid | 0,2 |
| Vinylpyrrolidon/Vinylacetat-Copolymerisat (Luviskol^{R} VA64W) | 2,0 |
| Konservierungsmittel (Parabene) | 0,5 |
| Trilaureth-4-phosphat | 3,0 |
| Parfum | 0,3 |
| Wasser | ad 100,0 |

Es wurde eine exzellente haarkonditionierende Wirksamkeit im Hinblick auf einen vollen Griff und Volumen, Spannkraft und dezenten Glanz erreicht.

## Patentansprüche

1. Haarbehandlungsmittel in Form einer Öl-in-Wasser-Emulsion, enthaltend
a) mindestens ein anionisches, nichtionisches, kationisches und/oder zwitterionisches bzw. amphoteres Polymer, und
b) ein Gemisch aus einem Salz eines Acrylsäure-Polymeren, einem C₃-C₁₀-Alkyl-C₁₂-C₁₈-fettsäureester und einem C₁₂-C₂₄-Fettalkoholethoxylat ausgewählt aus Trideceth-3, Trideceth-6, Laureth-4, Laureth-8, Myristeth-4, Myristeth-6, Ceteth-2 und Oleth-5.

2. Mittel nach Anspruch 1, enthaltend etwa 0,1 bis 5 Gew.-% des Bestandteils a), berechnet auf die Gesamtzusammensetzung.

3. Mittel nach Anspruch 1 oder 2, enthaltend als Bestandteil a) ein nichtionisches Polymer.

4. Mittel nach einem oder mehreren der Ansprüche 1 bis 3, enthaltend etwa 0,1 bis etwa 5 Gew.-% des Bestandteils b), berechnet auf die Gesamtzusammensetzung.

5. Mittel nach einem oder mehreren der Ansprüche 1 bis 4, enthaltend als Bestandteil b) ein Gemisch aus einem Alkalisalz eines Acrylsäure-Polymeren, Ethylhexylstearat und einem Tridecylalkoholethoxylat.

## Claims

1. Hair treatment composition in form of an oil-in-water emulsion, comprising
a) at least one anionic, nonionic, cationic and/or zwitterionic or amphoteric polymer, and
b) a mixture of a salt of an acrylic acid polymer, a C₃-C₁₀-alkyl-C₁₂-C₁₈-fatty acid ester and a C₁₂-C₂₄-fatty alcohol ethoxylate, selected from Trideceth-3, Trideceth-6, Laureth-4, Laureth-8, Myristeth-4, Myristeth-6, Ceteth-2 and Oleth-5.

2. Composition according to claim 1, comprising about 0.1 % to 5 % by weight of component a), calculated to the total composition.

3. Composition according to claim 1 or 2, comprising a nonionic polymer as component a).

4. Composition according to one or more of claims 1 to 3, comprising about 0.1 % to about 5 % by weight of component b), calculated to the total composition.

5. Composition according to one or more of claims 1 to 4, comprising as component b) a mixture of an alkali salt of an acrylic acid polymer, ethyl hexyl stearate and a tridecyl alcohol ethoxylate.

## Revendications

1. Agent de traitement de cheveux sous la forme d'une émulsion d'huile dans l'eau comprenant :
a) au moins un polymère anionique, non ionique, cationique et/ou zwitterionique ou amphotère, et
b) un mélange d'un sel d'un polymère d'acide arcylique, d'un (C₃-C₁₀) alkyl-ester d'acide gras en C₁₂ à C₁₈ et d'un éthoxylate d'alcool gras en C₁₂ à C₂₄, sélectionné parmi le tridéceth-3, le tridéceth-6, le laureth-4, le laureth-8, le myristeth-4, le myristeth-6, le ceteth-2 et l'oleth-5.

2. Agent selon la revendication 1, qui comprend entre environ 0,1 et 5 % en poids de l'élément a) par rapport à la composition totale.

3. Agent selon la revendication 1 ou 2, qui comprend un polymère non ionique comme élément a).

4. Agent selon l'une ou plusieurs des revendications 1 à 3, qui comprend entre environ 0,1 et environ 5 % en poids de l'élément b) par rapport à la composition totale.

5. Agent selon l'une ou plusieurs des revendications 1 à 4. qui comprend comme élément b) un mélange composé d'un sel de métal alcalin d'un polymère d'acide acrylique, de stéarate d'éthylhexyle et d'un éthoxylate d'alcool tridécylique.
